# EUROPEAN PATENT APPLICATION

(11) **EP 2 335 734 A2**
(43) Date of publication of application: **22.06.2011**
(21) Application number: 11157644.3
(22) Date of filing: 19.08.2004
(51) Int. Cl.: A61K 45/06, A61K 31/4168, A61K 31/498, A61K 31/135, A61K 31/517, A61K 31/506, A61P 29/00

(54) **Treatment of pain and other alpha 2 adrenergic-mediated conditions**

(30) Priority: 12.09.2003 US 502327 P; 30.06.2004 US 881761
(62) Divisional of application: 04781993.3
(71) Applicant: Allergan, Inc., Irvine, CA 92612 (US)
(72) Inventor: Donello, John E., Dana Point, CA 92629 (US); Gil, Daniel W., Corona Del Mar, CA 92625 (US)
(74) Representative: HOFFMANN EITLE

(57) **Abstract**

The present invention provides the following components a) and b) for use in the treatment or prevention of pain in a mammal by co-administration of the components:
a) a first component comprising a compound whose activity results in direct or indirect stimulation of alpha 2 adrenergic receptor activity, and
b) a second component comprising an alpha 1 adrenergic receptor antagonist;

wherein the dosage of the first component necessary to provide a given level of analgesia is less than that for a similarly affected mammal administered with the first component as the sole analgesic agent, or wherein the amount of sedation caused by the administration of a dose of the first component effective to cause half maximal analgesia is less than that caused in a similarly affected mammal administered with the first component in a dose effective to cause half maximal analgesia in the absence of the second component.

## Description

### BACKGROUND OF THE INVENTION

Human adrenergic receptors are integral membrane proteins which have been classified into two broad classes, the alpha and the beta adrenergic receptors. Both types mediate the action of the peripheral sympathetic nervous system upon binding of catecholamines, norepinephrine and epinephrine.

Norepinephrine is produced by adrenergic nerve endings, while epinephrine is produced by the adrenal medulla. The binding affinity of adrenergic receptors for these compounds forms one basis of the classification: alpha receptors tend to bind norepinephrine more strongly than epinephrine and much more strongly than the synthetic compound isoproterenol. The preferred binding affinity of these hormones is reversed for the beta receptors. In many tissues, the functional responses, such as smooth muscle contraction, induced by alpha receptor activation are opposed to responses induced by beta receptor binding.

Subsequently, the functional distinction between alpha and beta receptors was further highlighted and refined by the pharmacological characterization of these receptors from various animal and tissue sources. As a result, alpha and beta adrenergic receptors were further subdivided into three alpha 1, three alpha 2, and three beta subtypes.

Functional differences between alpha 1 and alpha 2 receptors have been recognized, and compounds which exhibit selective binding between these two subtypes have been developed. Thus, in WO 92/00073, the selective ability of the R(+) enantiomer of terazosin to selectively bind to adrenergic receptors of the alpha 1 subtypes was reported. The alpha 1/ alpha 2 selectivity of this compound was disclosed as being significant because agonist stimulation of the alpha 2 receptors was said to inhibit secretion of epinephrine and norepinephrine, while antagonism of the alpha 2 receptor was said to increase secretion of these hormones. Thus, the use of non-selective alpha-adrenergic blockers, such as phenoxybenzamine and phentolamine, was said to be limited by their α₂ adrenergic receptor mediated induction of increased plasma catecholamine concentration and the attendant physiological sequelae (increased heart rate and smooth muscle relaxation or contraction).

Additionally, a method for measuring alpha agonist activity and selectivity comprises the RESAT (Receptor Selection and Amplification Technology) assay as reported in Messier et al., High Throughout Assays of Cloned Adrenergic, Muscarinic, Neurokinin And Neurotrophin Receptors In Living Mammalian Cells, Pharmacol. Toxicol. 76:308-11 (1995) and adapted for use with alpha 2 receptors. The assay measures a receptor-mediated loss of contact inhibition that results in selective proliferation of receptor-containing cells in a mixed population of confluent cells. The increase in cell number is assessed with an appropriate transfected marker gene such as β-galactosidase, the activity of which can be easily measured in a 96-well format. Receptors that activate the G protein, Gq, elicit this response. Alpha₂ receptors, which normally couple to Gᵢ, activate the RSAT response when coexpressed with a hybrid Gq protein that has a Gi receptor recognition domain, called G_{q}/i5². See Conklin et al., Substitution Of Three Amino Acids Switches Receptor Specificity Of Gga To That Of Gia, Nature 363:274-6. (1993)

For a general background on the alpha-adrenergic receptors, the reader's attention is directed to Robert R. Ruffolo, Jr., α-Adrenoreceptors: Molecular Biology, Biochemistry and Pharmacology, (Progress in Basic and Clinical Pharmacology series, Karger, 1991), wherein. the basis of α₁/α₂ subclassification, the molecular biology, signal transduction, agonist structure-activity relationships, receptor functions, and therapeutic applications for compounds exhibiting α-adrenergic receptor affinity was explored.

The cloning, sequencing and expression of alpha receptor subtypes from human tissue sources has led to the subclassification of the alpha 1 adrenoreceptors into alpha 1A, alpha 1B, and alpha 1D. Similarly, the human alpha 2 adrenoreceptors have also been classified alpha 2A, alpha 2B, and alpha 2C receptors. Each alpha 2 receptor subtype appears to exhibit its own pharmacological and tissue specificities.

Alpha 2 receptor pan-agonists, such as clonidine and dexmedetomidine, have effective analgesic activity, and are currently generally administered directly to the central nervous system, e.g., intrathecally or epidurally for this purpose. Such alpha 2 receptor pan-agonists have sometimes been used for the treatment of chronic pain, such as cancer pain, post-operative pain, neuropathic pain, allodynia, post-herpetic neuralgia, irritable bowel syndrome, and other visceral pain. Other conditions in which such alpha 2 pan-agonists have been shown to have some therapeutic activity include addiction therapy (e.g., opiate or smoking detoxification), attention deficit-hyperactivity disorder (ADHD), Tourette's syndrome, depression and other psychiatric disorders, hypertension, ocular hypertension (such as that associated with some forms of glaucoma), and spasticity. However, these agents have not been commonly and effectively used as analgesic, agents or in the treatment of such other indications, due to a very narrow therapeutic window between the therapeutic effect and significant and sometimes overwhelming cardiovascular and sedative activity, as well as a significant interaction with other medications associated with the sedative effects. For examples of the latter, see e.g., Higuchi H., et al., The Interaction Between Propofol And Clonidine For Loss Of Consciousness, Anesth. Analg. 94(4): 886-91, (April 2002); Jaffe, R., et al., Adverse interaction between Clonidine and Verapamil, annals Pharmacother. 28(7-8) :881-3 (July-Aug 1994) (reporting on the potentially fatal synergism between sedative effects of clonidine and verapamil).

Because of common side effects including high sedative and cardiovascular depression activities at therapeutic doses, FDA approved alpha 2 receptor agonists (which to date have included only alpha 2 receptor pan-agonists) have generally been less useful as systemic agents than as local or topically-applied drugs. Thus, the alpha 2 pan-agonist clonidine has been used for the ophthalmologic treatment of high intraocular pressure (IOP) due to, for example, glaucoma. Since the drug is administered directly in the form of a drop to the eye, many of the usual systemic effects can be minimized. Nevertheless, even topical application of such drugs to the eye (which permits systemic delivery through osmosis into the blood vessels in the eye and through the nasolacrimal duct to the nose), does not eliminate these side effects, and thus therapeutically effective doses are still limited by such effects.

### SUMMARY OF THE INVENTION

In a first embodiment the present invention is related to the surprising discovery that coadministration to a mammal, particularly a human, of: 1) a first component comprising a compound whose activity results in a direct or indirect activation of the alpha 2 adrenergic receptor, and 2) a second component comprising an alpha 1 adrenergic receptor antagonist results in the increased potency of the therapeutic activity of the first component without significantly increasing the sedative activity thereof. Thus, the coadministration of the two components widens the therapeutic window between the therapeutic and sedative activities of the first component.

In a currently preferred embodiment, the invention is directed to a method for the treatment of a condition selected from the group consisting of pain, particularly chronic pain; neurodegenerative disorders; sympathetically-enhanced stress-associated conditions and ocular conditions including glaucoma and ocular hypertension.

Preferably, the compound comprised in the first component is selected from the group consisting of an alpha 2 receptor agonist and a norepinephrine transporter inhibitor (such as tricyclic antidepressants or TCA's). Examples of alpha 2 pan-agonists and TCA's have been well known in the art for years; however, the method disclosed herein is believed by the present inventors to be presented for the first time in this patent application.

The TCAs (which include amitriptyline (Amitril, Elavil) and nortriptyline (Aventyl, Pamelor); Desipramine (Pertofrane, Norpramin); Doxepin (Sinequan, Adapin); Imipramine (Janamine, Tofranil), Protriptyline (Vivactil); Trimipramine (Surmontil); and Clomipramine (Anafranil)) have been used first and foremost for the treatment of depressive conditions. However, they have been reported as useful in a wide range of disorders, such as major depressive episodes, some so-called atypical depression, panic disorder, social phobia, bulimia, narcolepsy, attention deficit disorder (ADD) with or without hyperactivity, migraine headache and various other chronic pain syndromes, including neuropathic pain, enuresis in children, and obsessive-compulsive disorder. Depressive symptoms occurring in the context of other major mental illnesses such as manic depressive disorder, schizophrenia and schizoaffective disorder, are also treated with TCAs, with certain caveats as discussed below.

With many TCA's, as with the alpha 2 receptor pan-agonists, a dangerous side effect is sedation. For this reason, they are generally prescribed to be administered at night, before sleep. Cardiovascular effects are also associated with these medications. Orthostatic hypotension, i.e. dizziness upon arising or otherwise rapidly changing posture, is common. A rapid heartbeat is often reported, sometimes with palpitations. The medications can have deleterious effects on an unhealthy heart, e.g. causing EKG (electrocardiogram) changes or arrhythmias (disturbance in cardiac rhythm or conduction) ; or, can worsen or precipitate angina or heart failure or a myocardial infarction (heart attack). These cardiac effects may eliminate the TCA's from consideration for many patients. It is the cardiac effects which make the tricyclics quite dangerous in overdose. An overdose can cause serious, potentially lethal, cardiac complications. Tricyclic antidepressants are now the leading cause of death by drug overdose in the United States.

The epidural administration of clonidine, an alpha 2 pan-agonist, has been reported to effectively provide pain relief and motor block during labor similar to those provided by the local anesthetics bupivacaine-fentanyl. Angelo, Reg. Anaesth. & Pain Med. 25:3 (Jan.-Feb. 2000), hereby incorporated by reference herein. However, these effects are accompanied by significantly more sedation and cardiovascular effects, such as hypotension, than was seen using the local anesthetics. Similar results are seen with other alpha 2 pan-agonists such as dexmeditomidine. Alpha 2 agonists, for example, alpha 2 receptor pan-agonists, have been used peripherally or non-peripherally for the treatment of chronic pain, such as cancer pain, post-operative pain, post-herpetic neuralgia, irritable bowel syndrome and other visceral pain, diabetic neuropathy, pain associated with muscle spasticity, complex regional pain syndrome (CRPS), sympathetically maintained pain, headache pain, allodynic pain, inflammatory pain, such as that associated with arthritis, gastrointestinal pain, such as irritable bowel syndrome (IBS) and Crohn's disease, and neuropathic pain. However, in each case, treatment with such a compound is limited by a narrow therapeutic window between analgesia on one hand and oversedation on the other.

Likewise, alpha 2 adrenergic agonists such as apraclonidine (a pan-agonist) and brimonidine have been used in ophthalmic formulations for the treatment of glaucoma and other ocular conditions involving high IOP or reduced uveo-scleral outflow of aqueous humor. While administration by installation of the drug directly into the eye reduces the systemic concentration of the drug, and thus the undesired side effects, some absorption or ingestion of the drug does nevertheless occur via installation. Therefore often the dose of drug necessary to most effectively treat high intraocular pressure is limited by the fact that deleterious side effects may also be seen at such concentrations.

By contrast, the present invention embraces methods for treating a mammal, including a human, having a condition responsive to treatment with an alpha 2 activating agent comprising the administration, to said mammal of an alpha 1 adrenergic receptor antagonist and an alpha 2 activating agent, wherein the degree of sedation or cardiovascular depression is less than is present following administration of an equivalently effective dose of the A2AA alone.

An "alpha 2 activating agent" or "A2AA" means an alpha 2 agonist, TCA, or other compound whose activity results in a direct or indirect activation of the alpha 2 adrenergic receptors.

Notably, though not exclusively, when used to treat pain, particularly chronic pain, the present method provides better analgesic activity (i.e. lower EC50) than in compositions comprising the A2AA as the sole analgesic agent, Moreover, such co-administration does not appear to substantially affect the dose-response relationship between the drug concentration and the sedative and hypotensive activity usually seen in compositions comprising the A2AA as the sole therapeutic agent. By "EC50" is meant the concentration of a given agent at which half the maximal measured activity is observed.

In analgesic and other applications, preferred routes of administration for the A2AA may be peripheral or non-peripheral and include oral, intravenous, intrathecal and epidural administration. Other possible means of administration of either component (or both components) include, without limitation, by intrathecal pump, subcutaneous pump, dermal patch, intravenous injection, subcutaneous injection, intramuscular injection, topical cream or gel, or an oral pill, or a combination of such methods. While peripheral means of administration of the A2AA are not currently preferred in certain applications, the advantages of the instantly claimed methods may be observed in such cases as well, depending at least in part on the nature of the agent and the indication for which it is administered.

In addition to the active ingredients, the first and second components preferably contain one or more pharmaceutically acceptable carrier consistent with the mode of administration chosen. The phrase "pharmaceutically-acceptable carrier" as used herein means a pharmaceutically-acceptable material, composition or vehicle, such as a liquid or solid filler, diluent, excipient, solvent or encapsulating material. Each carrier must be "acceptable" in the sense of being compatible with the other ingredients of the formulation, with the mode of administration, and not injurious to the patient. Some examples of materials which can serve as pharmaceutically-acceptable carriers include, without limitation: (a) sugars, such as lactose, glucose and sucrose; (b) starches, such as corn starch and potato starch; (c) cellulose, and its derivatives, such as sodium carboxymethyl cellulose, ethyl cellulose and cellulose acetate; (d) powdered tragacanth; (e) malt; (f) gelatin; (g) talc; (h) excipients, such as cocoa butter and suppository waxes; (i) oils, such as peanut oil, cottonseed oil, safflower oil, sesame oil, olive oil, corn oil and soybean oil; (j) glycols, such as propylene glycol; (k) polyols, such as glycerin, sorbitol, mannitol and polyethylene glycol; (1) esters, such as ethyl oleate and ethyl laurate; (m) agar; (n) buffering agents, such as magnesium hydroxide, aluminum hydroxide, boric acid and sodium borate, and phosphate buffers,; (o) alginic acid; (p) pyrogen-free watery; (q) isotonic saline; (r) Ringer's solution; (s) ethyl alcohol; (t) phosphate buffer solutions; and (u) other non-toxic compatible substances suitable for use in pharmaceutical formulations.

In methods for the treatment of chronic pain, the following may be helpful as an aid in the understanding of the invention. It is known that chronic pain (such as pain from cancer, arthritis, and many neuropathic injuries) and acute pain (such as that pain produced by an immediate mechanical stimulus, such as tissue section, pinch, prick, or crush) are distinct neurological phenomena mediated to a large degree either by different nerve fibers and neuroreceptors or by a rearrangement or alteration of the function of these nerves upon chronic stimulation. Sensation of acute pain is transmitted quite quickly, primarily by afferent nerve fibers termed C fibers, which normally have a high threshold for mechanical, thermal, and chemical stimulation. While the mechanisms of chronic pain are not completely understood, acute tissue injury can give rise within minutes or hours after the initial stimulation to secondary symptoms, including a regional reduction in the magnitude of the stimulus necessary to elicit a pain response. This phenomenon, which typically occurs in a region emanating from (but larger than) the site of the original stimulus, is termed hyperalgesia. The secondary response can give rise to profoundly enhanced sensitivity to mechanical or thermal stimulus.

The A afferent fibers (Aβ and Aδ fibers) can be stimulated at a lower threshold than C fibers, and appear to be involved in the sensation of chronic pain. For example, under normal conditions, low threshold stimulation of these fibers (such as a light brush or tickling) is not painful. However, under certain conditions such as those following nerve injury or in the herpesvirus-mediated condition known as shingles the application of even such a light touch or the brush of clothing can be very painful. This condition is termed allodynia and appears to be mediated at least in part by Aβ afferent nerves. C fibers may also be involved in the sensation of chronic pain, but if so it appears clear that persistent firing of the neurons over time brings about some sort of change which now results in the sensation of chronic pain.

As used herein, the term "pain" encompasses both acute and chronic pain. As used herein, the term "acute pain" means immediate, generally high threshold, pain brought about by injury such as a cut, crush, burn, or by chemical stimulation such as that experienced upon exposure to capsaicin, the active ingredient in chili peppers. The term "chronic pain," as used herein, means pain other than acute pain and includes, without limitation, neuropathic pain, visceral pain, fibromyalgia pain, inflammatory pain, headache pain, muscle pain and referred pain. It is understood that chronic pain often is of relatively long duration, for example, months or years and can be continuous or intermittent.

In one embodiment, the methods of the invention are used to treat "neuropathic pain," which, as used herein, means pain resulting from injury to a nerve. Neuropathic pain can be distinguished from nociceptive pain, which is pain caused by acute tissue injury involving small cutaneous nerves or small nerves in muscle or connective tissue. In contrast to neuropathic pain, nociceptive pain usually is limited in duration to the period of tissue repair and usually can be alleviated by available analgesic agents or opioids (Myers, Regional Anesthesia 20:173-184 (1995)).

Neuropathic pain typically is long-lasting or chronic and can develop days or months following an initial acute tissue injury. Neuropathic pain can involve persistent, spontaneous pain, as well as allodynia, which is a painful response to a stimulus that normally is not painful, or hyperalgesia, an accentuated response to a painful stimulus that usually is trivial, such as a pin prick. Neuropathic pain generally is resistant to opioid therapy (dyers, supra, 1995).

The methods of the invention are useful for treating neuropathic pain resulting from, without limitation, a trauma, injury or disease of peripheral nerve, dorsal root ganglia spinal cord, brainstem, thalamus or cortex. Examples of neuropathic pain that can be treated by the methods of the invention include neuralgia such as post-herpetic neuralgia, deafferentation pain and diabetic neuropathy. It is understood that the methods of the invention are useful in treating neuropathic pain regardless of the etiology of the pain. As non-limiting examples, the methods of the invention can be used to treat neuropathic pain resulting from a peripheral nerve disorder such as neuroma; from nerve compression; from nerve crush or stretch or incomplete nerve transsection; or from a mono-neuropathy or polyneuropathy. As further non-limiting examples, the methods of the invention are useful in treating neuropathic pain resulting from a disorder such as dorsal root ganglion compression; inflammation of the spinal cord; contusion, tumor or hemisection of the spinal cord; and tumors or trauma of the brainstem, thalamus or cortex.

As indicated above, the methods of the invention can be useful for treating neuropathic pain resulting from a mononeuropathy or polyneuropathy. A neuropathy is a functional disturbance or pathological change in the peripheral nervous system and is characterized clinically by sensory or motor neuron abnormalities. The term mononeuropathy indicates that a single peripheral nerve is affected, while the term polyneuropathy indicates that several peripheral nerves are affected. The etiology of a neuropathy can be known or unknown. Known etiologies include complications of a disease or toxic state such as diabetes, which is the most common metabolic disorder causing neuropathy, or irradiation, ischemia or vasculitis. Polyneuropathies that can be treated by a method of the invention can result, without limitation, from post-polio syndrome, diabetes, alcohol, amyloid, toxins, HIV, hypothyroidism, uremia, vitamin deficiencies, chemotherapy, 2',3'-didexoycytidine (ddC) treatment or Fabry's disease. It is understood that the methods of the invention can be used to treat chronic pain of these or other chronic neuropathies of known or unknown etiology.

The methods of the invention also can be used for treating chronic pain resulting from headache, including tension-type headache, migraine headache, cluster headache, hormone headache, rebound headache, sinus headache, and organic headache. The methods of the invention further can be used for treating chronic pain resulting from activity, such as, as non-limiting examples, long hours of work at a computer, work with heavy objects or heavy machinery, or spending long hours on one's feet, and repetitive motion disorders (RMDs). RMDs are a variety of muscular conditions that can cause chronic pain. RMDs can be caused by overexertion, incorrect posture, muscle fatigue, compression of nerves or tissue, too many uninterrupted repetitions of an activity or motion, or friction caused by an unnatural or awkward motion such as twisting the arm or wrist. Common RMDs occur in the hands, wrists, elbows, shoulders, neck, back, hips, knees, feet, legs, and ankles, however, the hands and arms are most often affected. The methods of the invention can be used to treat chronic pain arising from any type of RMD.

The methods of the invention further can used for treating chronic pain resulting from excessive muscle tension, such as certain types of back pain, such as that resulting from a herniated disc; sciatica and joint pain, as well as chronic pain resulting from inflammation, including inflammation caused by an inflammatory disorder such as osteo- and rheumatoid arthritis; inflammation caused by injury, such as a crush, puncture, stretch of a tissue or joint; inflammation caused by infection, such as tuberculosis; or neurogenic inflammation. As non-limiting examples, the methods of the invention can be used to treat chronic gastrointestinal inflammation including Crohn's disease, ulcerative colitis, gastritis, irritable bowel disease and chronic visceral pain such as pain caused by cancer or attendant to the treatment of cancer, for example, attendant to chemotherapy or radiation therapy. Similarly, the methods of the invention can be used to treat chronic inflammatory pain resulting, for example, from arthritis such as rheumatoid arthritis, gouty arthritis, or osteoarthritis; spondylitis; or autoimmune diseases such as lupus erythematosus. The methods of the invention further can be used to treat chronic muscle pain, chronic pain associated with substance abuse or withdrawal, and other types of chronic pain of known or unknown etiology. In the method of the present invention it is contemplated that the first and second drug may be coadministered using any relevant method. They may be administered using the same or different methods, and simultaneously or at different times.

In addition to the treatment of pain, alpha 2 agonists and TCAs are known to be useful for neuroprotection. For example, conditions which can be treated with the first and second components indicated herein which result in fewer side effects according to a method of the invention include, without limitation, neurodegenerative conditions such as Parkinson's disease, Alzheimer's disease, amyotrophic lateral sclerosis and multiple sclerosis; ischemia such as stroke; epilepsy; and neuropathies such as diabetic and ischemic retinopathy. Additionally, psychiatric conditions such as schizophrenia and bipolar disorder are now thought to involve neurodegeneration to some degree.

Additionally, the compositions and methods of the present invention are now known to be useful for the prevention or treatment of any of a variety of sympathetically-enhanced stress-associated conditions without concomitant sedation. Such conditions include, without limitation, sensory hypersensitivity, for example, sensory hypersensitivity associated with fibromyalgia or headache such as migraine; gastrointestinal diseases such as irritable bowel syndrome and dyspepsia; dermatological conditions such as psoriasis; cardiovascular disorders; tachycardias; disorders of peripheral vasoconstriction such as Raynaud's Syndrome and scleroderma; panic attack; metabolic disorders such as type II diabetes, insulin-resistance and obesity; disorders of muscle contraction including disorders of skeletal muscle contraction, disorders of smooth muscle contraction, spasticity, and disorders of muscle contraction associated with tension-type headache; behavioral disorders; and sexual dysfunction. In one embodiment, the sympathetically-enhanced condition is a condition other than sympathetically maintained pain, which is any pain that can be relieved by sympathetic blockade. Background information regarding such uses can be found in U.S. Patent Application NO. 60/502,840, entitled "NOVEL METHODS FOR IDENTIFYING IMPROVED, NON-SEDATING α-2 AGONISTS" filed on September 12, 2003, the same day as, and under a common obligation of assignment to the assignee of, this patent application, and hereby incorporated by reference herein.

Based on the coadministration of alpha 2 activating agents with alpha 1 antagonists, the present invention provides therapeutic efficacy in the treatment of neurodegenerative conditions with fewer sedative or cardiovascular side effects than would be seen using the A2AA alone.

By reducing or preventing neuronal death, an improvement in pathophysiology or symptoms can be appreciated. As used herein, the term "neuronal death" means destruction of a nerve cell resulting from induction of death in response to an insult or abnormality. Not included in the definition of "neuronal death" is non-pathological neuronal apoptosis, such as that which occurs during embryonic development or in self-renewing tissues containing apoptosis-liable neurons, such as the olfactory epithelium. Therefore, the term neuronal death can include non-olfactory neuroepithelial neuronal damage, such as damage of central nervous system neurons such as brain neurons and neuronal damage within nor-apoptosis-liable neurons. As used herein, the term "reducing," when used in reference to neuronal death means preventing, decreasing or eliminating the induction of death in a nerve cell. Reducing neuronal death by administering an effective amount of an A2AA and an alpha 1 antagonist can be an effective method for treating conditions involving neuronal death or dysfunction with minimized sedative or cardiovascular side effects.

As used herein, the term "neurodegenerative condition" means a disorder characterized by progressive nervous system dysfunction. Neurodegenerative conditions include a heterogeneous group of diseases of the central or peripheral nervous system that have many different etiologies. Such conditions can be, without limitation, hereditary, secondary to toxic or metabolic processes, and can result from infection. Neurodegenerative conditions are progressive conditions that can be age associated or chronic. Such conditions can be characterized by abnormalities of relatively specific regions of the brain or specific populations of neurons. The particular cell groups affected in different neurodegenerative conditions typically determine the clinical phenotype of the condition. In particular, neurodegenerative conditions can be associated with atrophy of a particular affected central or peripheral nervous system structure.

Exemplary neurodegenerative conditions include, but are not limited to, Motor Neuron Disease (ALS), Parkinsonian Syndromes, multiple sclerosis, diffuse cerebral cortical atrophy, Lewy-body dementia, Pick disease, mesolimbocortical dementia, thalamic degeneration, bulbar palsy, Huntington chorea, cortical-striatal-spinal degeneration, cortical-basal ganglionic degeneration, cerebrocerebellar degeneration, familial dementia with spastic paraparesis, polyglucosan body disease, Shy-Drager syndrome, olivopontocerebellar atrophy, progressive supranuclear palsy, dystonia musculorum deformans, Hallervorden-Spatz disease, Meige syndrome, familial tremors, Gilles de la Taurette syndrome, acanthocytic chorea, Friedreich ataxia, Holmes familial cortical cerebellar atrophy, AIDS related dementia, Gerstmann-Straussler-Scheinker disease, progressive spinal muscular atrophy, progressive balbar palsy, primary lateral sclerosis, hereditary muscular atrophy, spastic paraplegia, peroneal muscular atrophy, hypertrophic interstitial polyneuropathy, heredopathia atactica polyneuritiformis, optic neuropathy, diabetic retinopathy, Alzheimer's disease and ophthalmoplegia. The skilled person understands that these and other mild, moderate or severe neurodegenerative conditions can be treated according to a method of the invention.

In another embodiment of the invention are provided methods for the treatment of an ocular condition comprising coadministration of a first component comprising a A2AA, and a second component comprising an Alpha 1 receptor antagonist. In this embodiment therapeutic efficacy can be obtained at concentrations of the A2AA that result in greatly lowered sedative and cardiovascular effects.

Examples of ocular conditions that can be treated using a method of the invention include, but are not limited to, glaucoma, including open angle glaucoma, ocular hypertension, maculopathies and retinal degeneration, such as Non-Exudative Age Related Macular Degeneration (ARMD), Exudative Age Related Macular Degeneration (ARMD), Choroidal Neovascularization, Diabetic Retinopathy, Central Serous Chorioretinopathy, Cystoid Macular Edema, Diabetic Macular Edema, Myopic Retinal Degeneration; inflammatory diseases, such as Acute Multifocal Placoid Pigment Epitheliopathy, Behcet's Disease, Birdshot Retinochoroidopathy, Infectious (Syphilis, Lyme, Tuberculosis, Toxoplasmosis), Intermediate Uveitis (Pars Planitis), Multifocal Choroiditis, Multiple Evanescent White Dot Syndrome (MEWDS), Ocular Sarcoidosis, Posterior Scleritis, Serpiginous Choroiditis, Subretinal Fibrosis and Uveitis Syndrome, Vogt-Koyanagi-Harada Syndrome, Punctate Inner Choroidopathy, Acute Posterior Multifocal Placoid Pigment Epitheliopathy, Acute Retinal Pigement Epitheliitis, Acute Macular Neuroretinopathy; vascular and Exudative diseases, such as Diabetic retinopathy, Central Retinal Arterial Occlusive Disease, Central Retinal Vein Occlusion, Disseminated Intravascular Coagulopathy, Branch Retinal Vein Occlusion, Hypertensive Fundus Changes, Ocular Ischemic Syndrome, Retinal Arterial Microaneurysms, Coat's Disease, Parafoveal Telangiectasis, Hemi-Retinal Vein Occlusion, Papillophlebitis, Central Retinal Artery Occlusion, Branch Retinal Artery Occlusion, Carotid Artery Disease (CAD), Frosted Branch Angiitis, Sickle Cell Retinopathy and other Hemoglobinopathies, Angioid Streaks, Familial Exudative Vitreoretinopathy; Eales Disease; traumatic, surgical and environmental disorders, such as Sympathetic Ophthalmia, Uveitic Retinal Disease, Retinal Detachment, Trauma, Retinal Laser, Photodynamic therapy, Photocoagulation, Hypoperfusion During Surgery, Radiation Retinopathy, Bone Marrow Transplant Retinopathy; proliferative disorders, such as Proliferative Vitreal Retinopathy and Epiretinal Membranes; infectious disorders, such as Ocular Histoplasmosis, Ocular Toxocariasis, Presumed Ocular Histoplasmosis Syndrome (POHS), Endophthalmitis, Toxoplasmosis, Retinal Diseases Associated with HIV Infection, Choroidal Disease Associate with HIV Infection, Uveitic Disease Associate with HIV Infection, Viral Retinitis, Acute Retinal Necrosis, Progressive Outer Retinal Necrosis, Fungal Retinal Diseases, Ocular Syphilis, Ocular Tuberculosis, Diffuse Unilateral Subacute Neuroretinitis, Myiasis; genetic disorders, such as Retinitis Pigmentosa, Systemic Disorders with Accosiated Retinal Dystrophies, Congenital Stationary Night Blindness, Cone Dystrophies, Stargardt's Disease And Fundus Flavimaculatus, Best's Disease, Pattern Dystrophy of the Retinal Pigmented Epithelium, X-Linked Retinoschisis, Sorsby's Fundus Dystrophy, Benign Concentric Maculopathy, Bietti's Crystalline Dystrophy, pseudoxanthoma elasticum; retinal injuries, such as Macular Hole, Giant Retinal Tear; retinal tumors, such as Retinal Disease Associated With Tumors, Congenital Hypertrophy Of The RPE, Posterior Uveal Melanoma, Choroidal Hemangioma, Choroidal Osteoma, Choroidal Metastasis, Combined Hamartoma of the Retina and Retinal Pigmented Epithelium, Retinoblastama, Vasoproliferative Tumors of the Ocular Fundus, Retinal Astrocytoma, and Intraocular Lymphoid Tumors.

Ischemia of the neuroretina and optic nerve can arise during retinal branch vein occlusion, retinal branch artery occlusion, central retinal artery occlusion, central retinal vein occlusion, during intravitreal surgery, in retinal degenerations such as retinitis pigmentosa, and age-related macular degeneration.

The ability of the compositions used in the methods of the present invention, such as the coadministered A2AA and alpha1 antagonist, to reduce neuronal death or dysfunction can be assessed by analyzing an observable sign or symptom of nerve cell destruction in the presence and absence of treatment with the compound. Initiation of apoptotic death of neurons can have observable effects on cell function and morphology, as well as observable effects on tissues, organs and animals that contain dysfunctional or apoptotic neurons. Therefore, an indicator of neuronal damage can include observable parameters of molecular changes, such as increased expression of apoptosis-induced genes; cell function changes, such as reduced mitochondrial functions; cell morphological changes, such as cell shrinkage and blebbing; organ and tissue functional and morphological changes, such as the presence of an infarct or other lesion, the severity of which can be assessed by parameters including lesion volume and lesion size; physiological changes in animal models, including functional changes, such as loss of motor function, increased mortality and decreased survival, and behavioral changes, such as onset of dementia or loss of memory.

A reduction in an indicator of neuronal damage can be assessed in a cell, tissue, organ or animal by comparing an indicator of neuronal damage in at least two states of a cell, tissue, organ or animal. Thus, a reduction in an indicator of neuronal damage can be expressed relative to a control condition. A control condition can be, for example, a cell, tissue, organ or animal prior to treatment, in the absence of treatment, in the presence of a different treatment, in a normal animal or another condition determined to be appropriate by one skilled in the art.

In particular embodiments, a method of the invention is practiced by peripheral administration of the first and second component of the invention. As used herein, the term "peripheral administration" or "administered peripherally" means introducing an agent into a subject outside of the central nervous system. Peripheral administration encompasses any route of administration other than direct administration to the spine or brain. As such, it is clear that intrathecal and epidural administration as well as cranial injection or implantation, while within the scope of embodiments of the invention, are not within the scope of the terms "peripheral administration" or "administered peripherally." "

Peripheral administration can be local or systemic. Local administration results in significantly more of a pharmaceutical composition being delivered to the site of local administration than to regions distal to the site of administration. Systemic administration results in delivery of a pharmaceutical composition to essentially the entire peripheral nervous system of the subject and can also result in delivery to the central nervous system depending on the properties of the composition.

Routes of peripheral administration useful in the methods of the invention encompass, without limitation, oral administration, topical administration, intraocular administration, intravenous or other injection, and implanted minipumps or other extended release devices or formulations. A pharmaceutical composition useful in the invention can be peripherally administered, for example, orally in any acceptable form such as in a tablet, liquid, capsule, powder, or the like; by intravenous, intraperitoneal, intramuscular, subcutaneous or parenteral injection; by transdermal diffusion or electrophoresis; topically in any acceptable form such as in drops, creams, gels or ointments; and by minipump or other implanted extended release device or formulation.

The present invention also concerns methods for the treatment of pain in a mammal, comprising the coadministration, peripherally or non-peripherally of: a) a first component comprising a compound whose activity results in a direct or indirect activation of the alpha 2 adrenergic receptor, and b) a second component comprising an alpha 1 adrenergic receptor antagonist, wherein the amount of sedation caused by the administration of a dose of the first component effective to cause half maximal analgesia according to said method is less than that caused in a similarly affected mammal administered said first component in the absence of said second component, in a dose effective to cause half maximal analgesia. The mode of administration for this and other embodiments of the invention may be non-peripheral, e.g., intrathecal or epidural, or systemic, such as oral, intraperitoneal, intravenous, intramuscular, or transdermal.

In addition to the treatment of pain, another embodiment of the invention is drawn to methods for the treatment of other conditions for which alpha receptor agonists are known to be effective. These include, without limitation, ocular disorders such as ocular hypertension and glaucoma, sympathetically-enhanced stress-associated conditions, neurodegenerative conditions.; and spasticity. Treatment of these conditions with alpha adrenergics may also give rise to unwanted sedative side effects which the present invention can help ameliorate.

In this embodiment, topical delivery of the first and second components may be preferred for therapeutic delivery of the first and second components to the eye. Topical ophthalmic formulations are well known in the art.

Ophthalmic pharmaceutical compositions may be prepared by combining a therapeutically effective amount of the first and second component (either as a single formulation, or as separate formulations), as active ingredients, with conventional ophthalmically acceptable pharmaceutical excipients, and by preparation of unit dosage forms suitable for topical ocular use. The therapeutically efficient amount of each ingredient is typically between about 0.0001 and about 5% (w/w), preferably about 0.001 to about 1.0% (w/w) in liquid formulations.

For ophthalmic applications, preferably solutions may be prepared using a physiological saline solution as a major vehicle. The pH of such ophthalmic solutions should preferably be maintained between about pH 6.2 and pH 7.8 using an appropriate pharmaceutically acceptable buffer system, (such as, without limitation, a borate, tromethamine or phosphate buffer system). The formulations may also contain conventional, pharmaceutically acceptable preservatives, stabilizers and surfactants.

Preferred preservatives that may be used in the ophthalmic topical methods and compositions of the present invention include, but are not limited to, benzalkonium chloride, other polymeric quaternary ammonium preservatives (such as PHML and Polyquad®), chlorobutanol, thimerosal, phenylmercuric acetate and phenylmercuric nitrate. A particularly preferred class of preservatives are oxidative preservatives, such as stabilized chlorine dioxide, (e.g., Purite® stabilized chlorine dioxide), stabilized oxyborate, and the like.

Surfactants for use in an ophthalmic formulation may include ionic or non-ionic surfactants such as, without limitation, the Triton (e.g., Triton X-100), Tween® (e.g., polysorbate 40; polysorbate 80), and Pluronic® surfactants.

Likewise, various preferred vehicles may be used in the ophthalmic preparations of the present invention. These vehicles include, but are not limited to, polyvinyl alcohol; Carbopols® (polymers of propionic acid, cross-linked with allyl sucrose); Pemulin®; povidone; poloxamers; cellulosics, such as carboxymethyl cellulose, hydroxypropyl methylcellulose, hydroxyl methylcellulose and the like.

It may be preferable to formulate the ophthalmic formulation as an emulsion. If so, the emulsion may be either an oil-in-water emulsion or a water-in-oil emulsion. The emulsion may contain preservatives, and/or vehicles; however, the emulsion will usually contain at least one surfactant, and will contain an emulsifier. The emulsifier may also be a surfactant. One preferred emulsifier is Pemulin®, which is a cross-linked polyacrylate.

Tonicity adjustors may be added as needed or convenient. They include, but are not limited to, salts, particularly sodium chloride, potassium chloride, mannitol and glycerin, or any other suitable opthalmically acceptable tonicity adjuster.

Various buffers and means for adjusting pH may be used so long as the resulting preparation is ophthalmically acceptable. Accordingly, buffers include acetate buffers, citrate buffers, phosphate buffers and borate buffers. Acids or bases may be used to adjust the pH of these formulations as needed.

In a similar vein, a list of ophthalmically acceptable antioxidants for use in the present invention may include, but is not limited to, sodium metabisulfite, sodium thiosulfate, acetylcysteine, butylated hydroxyanisole and butylated hydroxytoluene.

Other excipient components which may be included in the ophthalmic preparations are chelating agents. The preferred chelating agent is edentate disodium, although other chelating agents may also be used in place or in conjunction with it.

The ingredients can be used in the following amounts:

| ingredient | amount (% w/w) |
|---|---|
| each active ingredient | about 0.001-5 |
| preservative | about 0-0.10 |
| vehicle | about 0-40 |
| tonicity adjustor | about 0-10 |
| buffer | about 0.01-10 |
| pH adjustor | Q.s. pH 4.5-7.5 |
| antioxidant | as needed |
| surfactant | as needed |
| purified water | Q.s. to 100% |

The actual dose of the active compounds of the present invention depends on the specific compound, and on the condition to be treated; the selection of the appropriate dose is well within the knowledge of the skilled artisan.

The ophthalmic formulations of the present invention are conveniently packaged in forms suitable for metered application, such as in containers equipped with a dropper, to facilitate application to the eye. Containers suitable for dropwise application are usually made of suitable inert, non-toxic plastic material, and generally contain between about 0.5 and about 15 ml solution.

Preservative-free solutions are often formulated in non-resealable containers containing up to about ten, preferably up to about five units doses, where a typical unit dose is from one to about 8 drops, preferably one to about 3 drops. The volume of one drop usually is about 20-35 .mu.l. In the methods of the present invention preferably the first component is selected from the group consisting of an alpha 2 agonist or a TCA; even more preferably an alpha 2 agonist; most preferably an alpha 2 pan-agonist selected from the group consisting of clonidine, brimonidine, dexmeditomidine, mivazerol and tizanidine. Such compounds and their syntheses are well-known.

In specific embodiments, the α1 adrenergic receptor antagonist is selected from the group consisting of prazosin and terazosin and 5-methylurapadil. The former two compounds and their syntheses are described in U.S. Patents 3,511,836, and 4,026,894, respectively; the latter compound is an easily synthesized derivative of urapidil, whose synthesis is described in U.S. Patent 3,957,786. These and all other references cited in this patent application are hereby incorporated by reference herein. Additionally, other alpha 1 receptor antagonists are well known in the art; many such compounds have been clinically approved. See also Lagu, 26 Drugs of the Future 757-765 (2001) and Forray et al., 8 Exp. Opin. Invest. Drugs 2073 (1999), hereby incorporated by reference herein, which provide examples of numerous alpha 1 antagonists.

Other embodiments will be apparent to one of skill in the art in light of the present specification.

### BRIEF DESCRIPTION OF THE DRAWINGS

Figure 1 shows analgesic activity of clonidine, either with or without co-administration of 5-methylurapadil, in wild type mice using the sulprostone-induced allodynia model.
Figure 2 is a dose response curve of clonidine sedative activity, either with or without coadministration of 5-methylurapadil, performed as described in greater detail below. The results show that 5-methylurapadil has no effect on clonidine sedation.
Figure 3 shows analgesic activity of amitriptylene, either with or without co-administration of 5-methylurapadil, in wild type mice using the sulprostone-induced allodynia model.

### DETAILED DESCRIPTION OF THE INVENTION

In a first embodiment, the present invention is related to the Applicants' surprising discovery that peripheral or, alternatively, non-peripheral coadministration to a mammal in need thereof of a composition comprising: a) a first component comprising a compound whose activity results in directly or indirectly stimulating alpha 2 adrenergic receptor activity, and b) a second component comprising an alpha 1 adrenergic receptor antagonist, results, when the dosage of the first component necessary to provide a therapeutic effect is less than that required to achieve a similar therapeutic effect in a similarly affected mammal administered said first component as a sole therapeutic agent, in lower sedation and/or cardiovascular side effects. This means that therapeutically effective effects, such as analgesia, ocular hypotensive activity, neuroprotection and the like may be obtained by co-administration of the first and second component in mammals, including humans, resulting in greatly decreased sedation and cardiovascular depression at a therapeutic dose as compared to administration of a similarly effective therapeutic dose of the first component. In a presently preferred embodiment, the mammal is in need of an analgesic due to chronic pain.

In this embodiment of the invention, therefore, the Applicants have discovered that the choadministration of a compound whose activity results in a direct or indirect activation of the alpha 2 adrenergic receptor (A2AA) e.g., an α2 receptor pan-agonist such as clonidine, dexmeditomidine, or tizanidine or a TCA, with an alpha 1 receptor antagonist results in an "unmasking" or enhancement of the alpha 2-mediated therapeutic activity of the A2AA with a concomitant increase in the therapeutic potency of the A2AA without a substantial increase in the sedative side effects commonly seen upon the use of such agents. As a result, the therapeutic window for the treatment with the selected A2AA is increased as compared to administration of the same drug without the alpha 1 antagonist, permitting, in certain cases, the use of increased doses of the A2AA, and in other cases the use of the same dose with fewer side effects. Preferably, the A2AA is an alpha 2 adrenergic receptor agonist.

While the invention is not to be construed as being limited by any particular theory, Applicants believe the invention functions because stimulation of the alpha 1 receptor(s), or one or more of the subtypes thereof, comprising the alpha 1A, alpha 1B and alpha 1D receptor in humans, causes an attenuation of the (e.g., analgesic, ocular hypotensive, neuroprotective, or other) activity resulting from a stimulation of the alpha 2A, alpha 2B, and/or alpha 2C receptor(s). It is also believed that the majority of A2AA's have not been determined to lack alpha 1 stimulatory activity (regardless whether described as being alpha 2 "selective" or not), and therefore possess sufficient intrinsic alpha 1 agonist activity to cause this attentuation effect.

Thus, it is believed that co-administration of an alpha 1 antagonist blocks the undesired opposing effects caused by stimulation of the alpha 1 receptor. Preferably the alpha 1 antagonist has at least alpha 1A receptor antagonist activity, alpha 1B antagonist activity or alpha 1D receptor antagonist activity. Most preferably, the alpha 1 receptor antagonist has at least alpha 1A receptor antagonist activity. Such antagonist may have antagonist activity at more than one alpha 1 receptor subtype.

The co-administration to a mammal of a A2AA and an alpha 1 antagonist, in addition to not increasing and sometimes reducing the sedative side effects seen upon treatment of a mammal with an alpha 2 agonist, also improves the therapeutic effect of the A2AA, (i.e., widens the "therapeutic window") thereby permitting treatment at lower doses than was otherwise possible.

In another embodiment, the present invention concerns a method for the treatment of pain in a mammal by administration of 1) a first component whose activity results in a direct or indirect activation of the alpha 2 adrenergic receptor, and 2) a second component comprising an alpha 1 adrenergic receptor antagonist. Preferably, the A2AA is selected from the group consisting of an alpha 2 receptor agonist and a norepinephrine transporter inhibitor (such as tricyclic antidepressants or TCA's); more preferably from alpha receptor agonists. Alternatively, the A2AA is preferably selected from the group consisting of brimonidine, clonidine, tizanidine, dexemedetomidine and norepinephrine and MPV-2426 (radolmidine).

Further, in another embodiment the invention involves methods for the treatment of high intraocular pressure in a mammal comprising administration of 1) a first component whose activity results in a direct or indirect activation of the alpha 2 adrenergic receptor, and 2) a second component comprising an alpha 1 adrenergic receptor antagonist. In this embodiment, the more favored route of administration may be a topical ophthalmic formulation, such as solution, suspension or emulsion. In a preferred embodiment, the A2AA is an alpha 2 agonist, more preferably, an alpha 2 pan-agonist.

Preferably the first and second components are contained in a single formulation for coadministration, although the first and second components may be co-administered as separate compositions. As used herein, the word "coadminister(ed)" shall include administration of the first and second component either as a single composition, or as separate compositions. Moreover, each of the components may be administered by a different route, and/or at the same or somewhat different times.

In an additional embodiment, the present invention encompasses compositions comprising a therapeutically effective dose of the first and second component in formulation with a pharmaceutically acceptable carrier. In a further embodiment, said first and second component may each be a domain of a single molecule.

For example, the first and second component may be linked by conjugation of an AA2A (selected from a TCA or an alpha 2 agonist) and an alpha 1 antagonist using a chemical linker, such as a bifunctional reagent.

Applicants now present examples for the purpose of illustrating certain embodiment of the invention. The invention is not intended to be limited by these examples.

### Example 1: Alleviation of Chronic Pain with Coadministered Alpha 2 Agonist and Alpha 1 Antagonist

A model for chronic pain (in particular peripheral neuropathy) involves the surgical ligation of the L5 (and optionally the L6) spinal nerves on one side in experimental animals. Rats recovering from the surgery gain weight and display a level of general activity similar to that of normal rats. However, these rats develop abnormalities of the foot, wherein the hindpaw is moderately everted and the toes are held together. More importantly, the hindpaw on the side affected by the surgery appears to become sensitive to pain from low-threshold mechanical stimuli, such as that producing a faint sensation of touch in a human, within about 1 week following surgery. This sensitivity to normally non-painful touch is called "tactile allodynia," and lasts for at least two months. The response includes lifting the affected hindpaw to escape from the stimulus, licking the paw and holding it in the air for many seconds. None of these responses is normally seen in the control group.

Rats are anesthetized before surgery. The surgical site is shaved and prepared either with betadine or novacaine. Incision is made from the thoracic vertebra XIII down toward the sacrum. Muscle tissue is separated from the spinal vertebra (left side) at the L4 - S2 levels. The L6 vertebra is located and the transverse process is carefully removed with a small rongeur to expose the L4 - L6 spinal nerves. The L5 and L6 spinal nerves are isolated and tightly ligated with 6-0 silk thread. The same procedure is done on the right side as a control, except no ligation of the spinal nerves is performed.

A complete hemostasis is confirmed, then the wounds are sutured. A small amount of antibiotic ointment is applied to the incised area, and the rat is transferred to the recovery plastic cage under a regulated heat-temperature lamp. On the day of the experiment, at least seven days after the surgery, six rats per test group are administered the test drugs by intrathecal injection, intraperitoneal injection, oral gavage or a combination of one or more of these.

Alternatively, allodynia can be induced in mice through intrathecal treatment of the animals witch 200nag sulprostone (prostaglandin E2 receptor agonist) in 50% DMSO and in a volume of 5µl. In this model, the pain response to stroking the flank with a paint brush is scored 8 times over a 35 minute period starting 15 minutes following spinal administration of sulprostone. Minami et al., 57 Pain 217-223 (1994). Sulprostone treatment alone elicits a score of 12-13 on a 16-point scale.

The compounds are formulated in approximately 0.01 - 5% DMSO and given in a volume of 1 ml/kg body weight for systemic dosing or 5 µl in saline for intrathecal dosing. Clozzidine is tested at intrathecal doses ranging between 0.01 and 10.0 µg.

In the Chung rat model, tactile allodynia is measured prior to and 30 minutes after drug administration using von Frey hairs that are a series of fine hairs with incremental differences in stiffness. Rats are placed in a plastic cage with a wire mesh bottom and allowed to acclimate for approximately 30 minutes. The van Frey hairs are applied perpendicularly through the mesh to the midplantar region of the rats' hindpaw with sufficient force to cause slight buckling and held for 6-8 seconds. The applied force has been calculated to range from 0.41 to 15.1 grams. If the paw is sharply withdrawn, it is considered a positive response. A normal animal will not respond to stimuli in this range, but a surgically ligated paw will be withdrawn in response to a 1-2 gram hair. The 50% paw withdrawal threshold is determined using the method of Dixon, W.J., Ann. Rev. Pharmacol. Toxicol. 20:441-462 (1980), incorporated by reference herein. The post-drug threshold is compared to the pre-drug threshold and the percent reversal of tactile sensitivity is calculated based on a normal threshold of 15.1 grams.

The results show that clonidine is analgesic in both rats and mice in a dose-dependent fashion. In the Chung model of allodynic rats there is no analgesia at an intrathecal dose of 0.1 µg, while maximal analgesia is observed at 1.0 µg. To determine the therapeutic window, rats are treated at various doses of clonidine and assayed for sedation.

To test sedation, six male Sprague-Dawley rats are given various doses of a clonidine intrathecal injection. Sedation is graded 30 minutes following administration of the drug by monitoring locomotor skills as follows. Rats are placed in a dark covered chamber and a digicom analyzer (Omnitech Electronic) quantitates their exploratory behavior for a five-minute period. The machine records each time the rat interrupts an array of 32 photoelectric beams in the X and Y orientation, and quantifies the difference in behavior as compared to control animals given saline instead of clonidine.

In this assay, an intrathecal dose of 3.0 µg is mildly sedating, while a dose of 10 µg induces maximal sedation. Thus, the separation between analgesia and sedation is approximately 3-10 fold.

In a mouse model of sulprostone-induced allodynia, similar results are obtained.

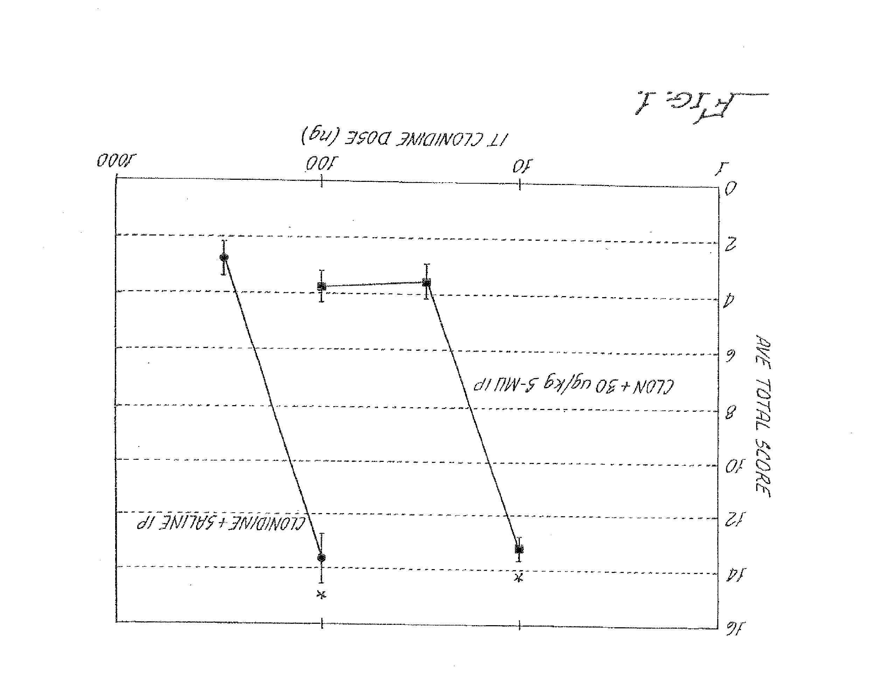

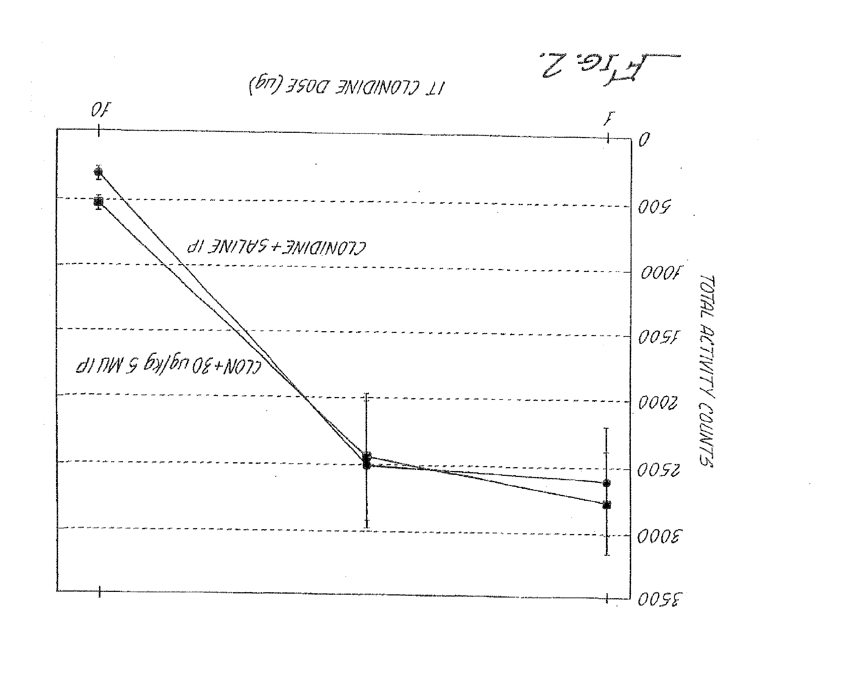

As shown in Figure 1, in the sulprostone-induced mouse model intraperitoneal administration of the alpha 1 antagonist 5-methyl urapidil at a dose of 30 µg/kg 5 minutes before administration of the clonidine shifts the effective analgesic dose 10-fold lower than the dose response using clonidine alone (see Figure 1), while leaving the sedating dose unchanged (See Figure 2). Thus, co-administration of clonidine (an alpha 2 agonist) and an alpha 1 antagonist causes a "widening" of the therapeutic window between pain and sedation from 3-10 fold to approximately 30-100 fold in a model of chronic pain.

### Example 2: Alleviation of Chronic Pain with Coadministered TCA and Alpha 1 Antagonist (5-methylurapadil).

Tricyclic antidepressants (TCAs), a commonly prescribed antidepressant and analgesic, indirectly stimulates the alpha 2 receptors by inhibiting norepinephrine uptake.

Experiments carried out in a manner similar to those described in Example 1 above are performed using the sulprostone-induced allodynic mouse models and the TCA amitriptylene. This compound and its synthesis are described in U.S. Patent 3,205,264, hereby incorporated by reference herein.

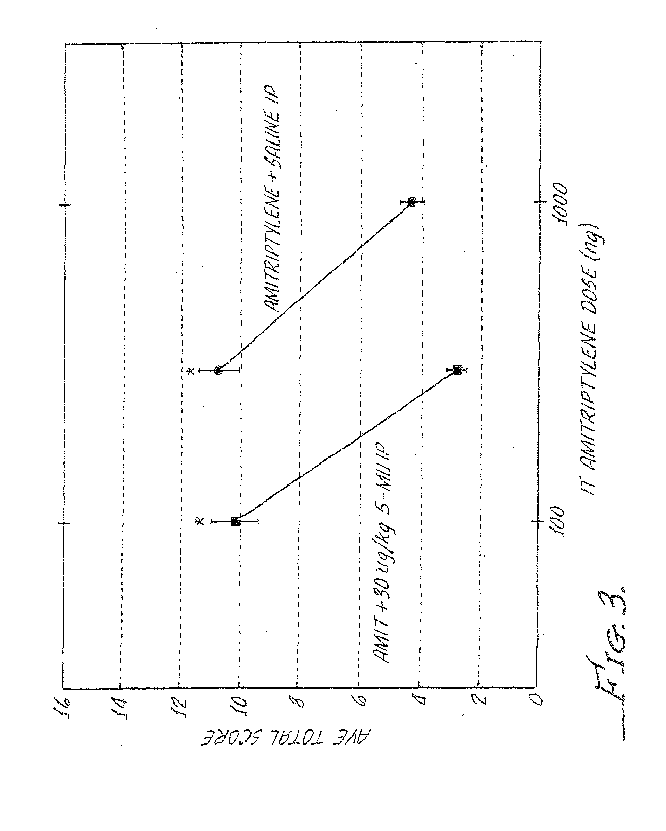

Amitriptylene is dissolved in 50% DMSO at the indicated doses and injected in a volume of 5 ul intrathecally into each mouse, in conjunction with either a 30 ug/kg IP injection of 5-methylurapadil or with a similar injection of saline. Paint brush stimulation as described in Example 1 was scored, and the results are shown in Figure 3. As with the combination of alpha 2 agonist and alpha 1 antagonist, amitriplylene in combination with an alpha 1 antagonist resulted in a reduction in the dosage necessary to achieve maximal analgesia, in this case, approximately three fold.

This example thus shows that directly or indirectly (e.g., either by increasing norepinephrine production or by limiting norepinephrine uptake or turnover) stimulating the alpha 2 adrenergic receptors, in conjunction with alpha 1 antagonism gives rise to the observed effect of increasing the therapeutic window between sedation and therapeutic efficacy as compared to the use of the A2AA alone.

### Example 3: Alleviation of Chronic Pain with Coadministered, Alpha 2 Agonist and Alpha 1 Antagonist (prazosin).

Using methodology similar to that described for the sulprostone-induced allodynia model, an intraperitoneal dose of the alpha 1 antagonist prazosin (100 ng/kg) has no effect on its own (pain score = 4.8 ± 0.6) or in the sulprostone-induced allodynia model (12.8 ± 0.8).

Prazosin is administered to mice 15 minutes before administration of the sulprostone and various intrathecal doses of clonidine (0.03, 0.1 and 0.4 micrograms in a 5 microliter volume of 50% DMSO). The clonidine dose response for analgesia upon coadministration of the alpha 1 antagonist is as follows: 13.3 ± 0.9 for the 0.03 microgram dose, 4.8 ± 0.8 for the 0.1 microgram dose, and 4.8 ± 0.6 for the 0.4 microgram dose. This represents approximately a four-fold decrease in the EC₅₀ for clonidine as compared to i.t. administration of clonidine alone.

Thus, the administration of both the A2AA and alpha 1 antagonist again resulted in an increase in the potency of the A2AA as compared to its use alone. In this experiment the 0.1 microgram dose of clonidine is not analgesic in the absence of prazosin.

### Example 4: Alleviation of Chronic Pain with Alpha 2 Agonist and Alpha 1 Antagonist Administered by the Same Route

Experimental procedure is the same as in Example 1, except both clonidine (various doses) and 5-methylurapidil (1 microgram intrathecal) are coadministered by intrathecal injection in a 50% DMSO vehicle and in a total volume of 5 microliters. Results are substantially similar to those seen upon intraperitoneal injection of the alpha 1 antagonist. Thus, the two agents can be administered together or at slightly different times, and may be administered by the same or different routes of administration, with the same or similar therapeutic effect.

The following are particular embodiments of the invention.
1) A composition for the treatment or prevention of a condition selected from the group consisting of pain, an ocular condition, a neurodegenerative condition, and a sympathetically-enhanced stress-associated condition, comprising:
   a) a first component comprising a compound whose activity results in a direct or indirect activation of the alpha 2 adrenergic receptor, and
   b) a second component comprising an alpha 1 adrenergic receptor antagonist, in a pharmaceutically acceptable carrier.
2) The composition of 1 in which the compound and the alpha 1 adrenergic receptor antagonist are not covalently linked.
3) The composition of 1 in which the compound and the alpha 1 adrenergic receptor antagonist are covalently linked.
4) A method for the treatment or prevention of a neurodegenerative condition in a mammal, comprising the coadministration of:
   a) a first component comprising a compound whose activity results in a direct or indirect activation of the alpha 2 adrenergic receptor, and
   b) a second component comprising an alpha 1 adrenergic receptor antagonist,
   wherein the amount of sedation caused by the administration a dose of the first component effective to cause half maximal neuroprotection according to said method is less than that caused in a similarly affected mammal administered said first component in the absence of said second component, in a dose effective to cause half maximal neuroprotection.
5) The method of 4 wherein said neurodegenerative condition is selected from the group consisting of Motor Neuron Disease (ALS), Parkinsonian Syndromes, multiple sclerosis, diffuse cerebral cortical atrophy, Lewy-body dementia, Pick disease, mesolimbocortical dementia, thalamic degeneration, bulbar palsy, Huntington chorea, cortical-striatal-spinal degeneration, cortical-basal ganglionic degeneration, cerebrocerebellar degeneration, familial dementia with spastic paraparesis, polyglucosan body disease, Shy-Drager syndrome, olivopontocerebellar atrophy, progressive supranuclear palsy, dystonia musculorum deformans, Hallervorden-Spatz disease, Meige syndrome, familial tremors, Gilles de la Tourette syndrome, acanthocytic chorea, Friedreich ataxia, Holmes familial cortical cerebellar atrophy, AIDS related dementia, Gerstmann-Straussler-Scheinker disease, progressive spinal muscular atrophy, progressive balbar palsy, primary lateral sclerosis, hereditary muscular atrophy, spastic paraplegia, peroneal muscular atrophy, hypertrophic interstitial polyneuropathy, heredopathia atactica polyneuritiformis, optic neuropathy, diabetic retinopathy, Alzheimer's disease and ophthalmoplegia.
6) The method of 4 wherein said first component comprises an alpha 2 adrenergic receptor agonist.
7) The method of 6 wherein said alpha 2 receptor agonist is an alpha 2 receptor pan-agonist.
8) The method of 6 wherein said alpha 2 receptor agonist is selected from the group consisting of brimonidine, clonidine, tizanidine, dexemedetomidine and mivazerol.
9) The method of 8 in which said alpha 2 adrenergic receptor agonist is brimonidine.
10) The method of 8 in which said alpha 2 adrenergic receptor agonist is clonidine.
11) The method of 8 in which said alpha 2 adrenergic receptor agonist is tizanidine.
12) The method of 8 in which said alpha 2 adrenergic receptor agonist is dexemedetomidine.
13) The method of 8 in which said alpha 2 adrenergic receptor agonist is mivazerol.
14) The method of 4 wherein said first component comprises a tricyclic antidepressant.
15) The method of 14 wherein said first component comprises amitriptylene.
16) The method of 4 wherein said alpha 1 adrenergic receptor antagonist is selected from the group consisting of 5-methylurapidil, urapidil, prazosin, bunazosin, terazosin, and doxazosin.
17) The method of 16 wherein said alpha adrenergic receptor antagonist is 5-methylurapidil.
18) The method of 16 wherein said alpha adrenergic receptor antagonist is urapidil.
19) The method of 16 wherein said alpha adrenergic receptor antagonist is prazosin.
20) The method of 16 wherein said alpha adrenergic receptor antagonist is terazosin.
21) The method of 16 wherein said alpha adrenergic receptor antagonist is doxazosin.
22) The method of 16 wherein said alpha adrenergic receptor antagonist is bunazosin.
23) A method for the treatment or prevention of an ocular condition in a mammal, comprising the coadministration of:
   a) a first component comprising a compound whose activity results in a direct or indirect activation of the alpha 2 adrenergic receptor, and
   b) a second component comprising an alpha 1 adrenergic receptor antagonist,
   wherein the amount of sedation caused by the administration a dose of the first component effective to cause half maximal therapeutic efficacy against said ocular condition according to said method is less than that caused in a similarly affected mammal administered said first component in the absence of said second component, in a dose effective to cause half maximal therapeutic efficacy against the same ocular condition.
24) The method of 23 wherein said condition is selected from the group consisting of: glaucoma, ocular hypertension, maculopathies, Non-Exudative Age Related Macular Degeneration (ARMD), Exudative Age Related Macular Degeneration (ARMD), Choroidal Neovascularization, Diabetic Retinopathy, Central Serous Chorioretinopathy, Cystoid Macular Edema, Diabetic Macular Edema, Myopic Retinal Degeneration; Acute Multifocal Placoid Pigment Epitheliopathy, Behcet's Disease, Birdshot Retinochoroidopathy, Intermediate Uveitis (Pars Planitis), Multifocal Choroiditis, Multiple Evanescent White Dot Syndrome (MEWDS), Ocular Sarcoidosis, Posterior Scleritis, Serpiginous Choroiditis, Subretinal Fibrosis, Uveitis Syndrome, Vogt-Koyanagi-Harada Syndrome, Punctate Inner Choroidopathy, Acute Posterior Multifocal Placoid Pigment Epitheliopathy, Acute Retinal Pigement Epitheliitis, Acute Macular Neuroretinopathy; Diabetic retinopathy, Central Retinal Arterial Occlusive Disease, Central Retinal Vein Occlusion, Disseminated Intravascular Coagulopathy, Branch Retinal Vein Occlusion, Hypertensive Fundus Changes, Ocular Ischemic Syndrome, Retinal Arterial Microaneurysms, Coat's Disease, Parafoveal Telangiectasis, Hemi-Retinal Vein Occlusion, Papillophlebitis, Central Retinal Artery Occlusion, Branch Retinal Artery Occlusion, Carotid Artery Disease (CAD), Frosted Branch Angiitis, Sickle Cell Retinopathy and other Hemoglobinopathies, Angioid Streaks, Familial Exudative Vitreoretinopathy; Eales Disease; Sympathetic Ophthalmia, Uveitic Retinal Disease, Retinal Detachment, Trauma, Retinal Laser, Photodynamic therapy, Photocoagulation, Hypoperfusion During Surgery, Radiation Retinopathy, Bone Marrow Transplant Retinopathy; Proliferative Vitreal Retinopathy, Epiretinal Membranes; Ocular Histoplasmosis, Ocular Toxocariasis, Presumed Ocular Histoplasmosis Syndrome (POHS), Endophthalmitis, Toxoplasmosis, Retinal Diseases Associated with HIV Infection, Choroidal Disease Associate with HIV Infection, Uveitic Disease Associate with HIV Infection, Viral Retinitis, Acute Retinal Necrosis, Progressive Outer Retinal Necrosis, Fungal Retinal Diseases, Ocular Syphilis, Ocular Tuberculosis, Diffuse Unilateral Subacute Neuroretinitis, Myiasis; Retinitis Pigmentosa, Systemic Disorders with Accosiated Retinal Dystrophies, Congenital Stationary Night Blindness, Cone Dystrophies, Stargardt's Disease And Fundus Flavimaculatus, Best's Disease, Pattern Dystrophy of the Retinal Pigmented Epithelium, X-Linked Retinoschisis, Sorsby's Fundus Dystrophy, Benign Concentric Maculopathy, Bietti's Crystalline Dystrophy, pseudoxanthoma elasticum; Macular Hole, Giant Retinal Tear; Retinal Disease Associated With Tumors, Congenital Hypertrophy Of The RPE, Posterior Uveal Melanoma, Choroidal Hemangioma, Choroidal Osteoma, Choroidal Metastasis, Combined Hamartoma of the Retina and Retinal Pigmented Epithelium, Retinoblastoma, Vasoproliferative Tumors of the Ocular Fundus, Retinal Astrocytoma, and Intraocular Lymphoid Tumors.
25) The method of 23 wherein said first component comprises an alpha 2 adrenergic receptor agonist.
26) The method of 25 wherein said alpha 2 receptor agonist is an alpha 2 receptor pan-agonist.
27) The method of 25 wherein said alpha 2 receptor agonist is selected from the group consisting of brimonidine, clonidine, tizanidine, dexemedetomidine and mivazerol.
28) The method of 27 in which said alpha 2 adrenergic receptor agonist is brimonidine.
29) The method of 27 in which said alpha 2 adrenergic receptor agonist is clonidine.
30) The method of 27 in which said alpha 2 adrenergic receptor agonist is tizanidine.
31) The method of 27 in which said alpha 2 adrenergic receptor agonist is dexemedetomidine.
32) The method of 27 in which said alpha 2 adrenergic receptor agonist is mivazerol.
33) The method of 23 wherein said first component comprises a tricyclic antidepressant.
34) The method of 33 wherein said first component comprises amitriptylene.
35) The method of 23 wherein said alpha 1 adrenergic receptor antagonist is selected from the group consisting of 5-methylurapidil, urapidil, prazosin, terazosin, bunazosin and doxazosin.
36) The method of 35 wherein said alpha adrenergic receptor antagonist is 5-methylurapidil.
37) The method of 35 wherein said alpha adrenergic receptor antagonist is urapidil.
38) The method of 35 wherein said alpha adrenergic receptor antagonist is prazosin.
39) The method of 35 wherein said alpha adrenergic receptor antagonist is terazosin.
40) The method of 35 wherein said alpha adrenergic receptor antagonist is doxazosin.
41) The method of 35 wherein said alpha adrenergic receptor antagonist is bunazosin.
42) The method of any one of 23, 25, 27, 33 and 35 in which the ocular condition is selected from the group consisting of glaucoma, ocular hypertension, macular degeneration, retinitis pigmentosa, and diabetic retinopathy.
43) A method for the treatment or prevention of an a sympathetically-enhanced stress-associated condition in a mammal, comprising the coadministration of:
   c) a first component comprising a compound whose activity results in a direct or indirect activation of the alpha 2 adrenergic receptor, and
   d) a second component comprising an alpha 1 adrenergic receptor antagonist,
   wherein the amount of sedation caused by the administration a dose of the first component effective to cause half maximal therapeutic efficacy against said sympathetically-enhanced stress-associated condition according to said method is less than that caused in a similarly affected mammal administered said first component in the absence of said second component, in a dose effective to cause half maximal therapeutic efficacy against the same sympathetically-enhanced stress-associated condition.
44) The method of 43 wherein said condition is selected from the group consisting of: sensory hypersensitivity; gastrointestinal diseases; dermatological conditions such as psoriasis; cardiovascular disorders; tachycardias; disorders of peripheral vasoconstriction; panic attack; metabolic disorders; insulin-resistance; and obesity; disorders of smooth muscle contraction; spasticity, and disorders of muscle contraction associated with tension-type headache; behavioral disorders; and sexual dysfunction.
45) The method of 43 wherein said first component comprises an alpha 2 adrenergic receptor agonist.
46) The method of 45 wherein said alpha 2 receptor agonist is an alpha 2 receptor pan-agonist.
47) The method of 45 wherein said alpha 2 receptor agonist is selected from the group consisting of brimonidine, clonidine, tizanidine, dexemedetomidine and mivazerol.
48) The method of 47 in which said alpha 2 adrenergic receptor agonist is brimonidine.
49) The method of 47 in which said alpha 2 adrenergic receptor agonist is clonidine.
50) The method of 47 in which said alpha 2 adrenergic receptor agonist is tizanidine.
51) The method of 47 in which said alpha 2 adrenergic receptor agonist is dexemedetomidine.
52) The method of 47 in which said alpha 2 adrenergic receptor agonist is mivazerol.
53) The method of 43 wherein said first component comprises a tricyclic antidepressant.
54) The method of 53 wherein said first component comprises amitriptylene.
55) The method of 43 wherein said alpha 1 adrenergic receptor antagonist is selected from the group consisting of 5-methylurapidil, urapidil, prazosin, terazosin, bunazosin and doxazosin.
56) The method of 55 wherein said alpha adrenergic receptor antagonist is 5-methylurapidil.
57) The method of 55 wherein said alpha adrenergic receptor antagonist is urapidil.
58) The method of 55 wherein said alpha adrenergic receptor antagonist is prazosin.
59) The method of 55 wherein said alpha adrenergic receptor antagonist is terazosin.
60) The method of 55 wherein said alpha adrenergic receptor antagonist is doxazosin.
61) The method of 55 wherein said alpha adrenergic receptor antagonist is bunazosin.
62) The method of any one of 43, 45, 47, 53 and 55 in which the sympathetically-enhanced condition is selected from the group consisting of disorders of muscle contraction associated with tension-type headache, sensory hypersensitivity, gastrointestinal diseases and psoriasis.

## Claims

1. The following components a) and b) for use in the treatment or prevention of pain in a mammal by coadministration of the components:
a) a first component comprising a compound whose activity results in direct or indirect stimulation of alpha 2 adrenergic receptor activity, and
b) a second component comprising an alpha 1 adrenergic receptor antagonist;
wherein the dosage of the first component necessary to provide a given level of analgesia is less than that for a similarly affected mammal administered with the first component as the sole analgesic agent.

2. The following components a) and b) for use in the treatment or prevention of pain in a mammal by coadministration of the components:
1) a first component comprising a compound whose activity results in direct or indirect activation of the alpha 2 adrenergic receptor, and
2) a second component comprising an alpha 1 adrenergic receptor antagonist;
wherein the amount of sedation caused by the administration of a dose of the first component effective to cause half maximal analgesia is less than that caused in a similarly affected mammal administered with the first component in a dose effective to cause half maximal analgesia in the absence of the second component.

3. Components for use according to claim 1 or claim 2, wherein the first component is administered directly to the central nervous system.

4. Components for use according to claim 1 or claim 2, wherein the first component is administered by peripheral or non-peripheral means.

5. Components for use according to claim 4, wherein the first component is administered by a method selected from intrathecal injection, intrathecal pump, subcutaneous pump, transdermal patch, intravenous injection, subcutaneous injection, intramuscular injection, topical cream or gel or oral administration.

6. Components for use according to claim 3, wherein the first component is administered intrathecally or epidurally.

7. Components for use according to claim 1 or claim 2, wherein the first component comprises an alpha 2 adrenergic receptor agonist.

8. Components for use according to claim 7, wherein the first component comprises an alpha 2 adrenergic receptor pan-agonist.

9. Components for use according to claim 7, wherein the alpha 2 adrenergic receptor agonist is selected from brimonidine, clonidine, tizanidine, dexemedetomidine and mivazerol.

10. Components for use according to claim 9, wherein the alpha 2 adrenergic receptor agonist is clonidine.

11. Components for use according to claim 1 or claim 2, wherein the first component comprises a tricyclic antidepressant.

12. Components for use according to claim 1 or claim 2, wherein the alpha 1 adrenergic receptor antagonist is selected from 5-methylurapidil, urapidil, prazosin, terazosin and doxazosin.

13. Components for use according to claim 1 or claim 2, wherein the first and second components are administered by the same route.
